# EUROPEAN PATENT APPLICATION

(11) **EP 1 275 309 A1**
(43) Date of publication of application: **15.01.2003**
(21) Application number: 02090232.6
(22) Date of filing: 05.07.2002
(51) Int. Cl.: A23L 1/30, A23C 9/152, A23D 9/013, A23D 7/00, C12P 7/64, C12N 9/20, C12P 7/62, C11C 3/00

(54) **Sterol fatty acid ester composition and foods containing the same**

(30) Priority: 13.07.2001 JP 2001214374; 26.04.2002 JP 2002127347
(71) Applicant: Ikeda Food Research Co. Ltd., Fukuyama-City, Hiroshima-ken 721-0956 (JP)
(72) Inventor: Mankura, Mitsumasa, Fukuyama-City, Hiroshima-ken 721-0956 (JP); Ikemoto, Masahiro, Fukuyama-City, Hiroshima-ken 721-0956 (JP); Sato, Fumi, c/o Ikeda Food Research Co., Ltd., Fukuyama-City, Hiroshima-ken 721-0956 (JP); Kaneko, Shoji, c/o Ikeda Food Research Co., Ltd., Fukuyama-City, Hiroshima-ken 721-0956 (JP); Seo, Naoko, c/o Ikeda Food Research Co., Ltd., Fukuyama-City, Hiroshima-ken 721-0956 (JP)
(74) Representative: Ziebig, Marlene, Dr. Dipl.-Chem.

(57) **Abstract**

The present invention provides foods containing sterol fatty acid esters composition and having pleasant texture, taste and flavor, wherein free sterols are hardly crystallized during storage, and a lot of the sterol fatty acid esters composition may be incorporated in foods having a low oil and fat content. The foods contain 1% by weight or more of the sterol fatty acid esters composition with a degree of esterification of more than 90%, thereby enabling foods containing oils and fats having a good emulsifying effect to be obtained.

## Description

### BACKGROUND OF THE INVENTION

### 1. Field of the Invention

The present invention relates to foods having pleasant texture, taste and flavor by allowing the foods to contain the sterol fatty acid ester composition having a degree of esterification of as high as more than 90%, whereby crystals are hardly formed during storage while enabling foods having a low proportion of oils and fats to contain a large quantity of the sterol fatty acid ester composition. In addition, the sterol fatty acid ester composition exhibits an emulsifying effect.

### 2. Description of the Related Art

Sterols such as β-sitosterol, which are obtained as a part of non-saponified products in the refining process of vegetable oils such as soybean oil and rape seed oil, have been known to have an action for lowering the blood serum cholesterol level.

However, since free sterols are hardly soluble in fats and oils. Recently, intake as sterol fatty acid esters have been proposed in order to improve solubility of sterols in oils and fats and to increase the amount of additive to foods containing oils and fats, and also the addition of the sterol fatty acid ester compositions into various foods such as margarine supplemented with sterol fatty acid esters has been attempted.

However, many sterols have been mixed with foods as their free forms only, or as a mixture of free sterols and sterol fatty acid esters (JP11-127779A, WO9638047 and WO9956558) Since free sterols have a solubility of as low as 1 to 2% in an oil, large free sterol crystals that significantly affect the quality of foods are formed in the foods. For example, crystallization of the free sterols is observed by adding about 3% of the free sterols in the foods. This problem is inevitable in the foods that require refrigeration for storing.

JP11-127779A discloses fat based foods containing at least 1% by weight of free sterol or esterified sterol with a degree of esterification of sterols within a range of 40 to 90%. However, crystallization of the free sterols or sterol fatty acid esters is observed during storage of the foods when the oils and fats contain a large proportion of the esterified sterols with a degree of esterification of the sterols of within a range of 40 to 90%. An additional heating and blending steps are required in the manufacturing process of the foods for melting the crystals and the manufacturing process becomes so complicated. Adding the heating and blending steps in the manufacturing process of the foods cause heat degradation such as degeneration of food components or oxidation of the food components in the foods containing the sterol fatty acid esters, thereby reducing market values.

Problems such as heterogeneous distribution of the food components, deterioration of the food texture such as rough feeling when eating, and poor appearance of the food products are caused by crystallization of the sterol fatty acid esters during storage, and these problems should be solved.

Recently, the content of oil and fat in foods containing oils and fats such as mayonnaise, dressing, ice cream and chocolate tend to be decreased in view of their effects on health. However, the amount of blend of the sterol and/or sterol fatty acid esters in the foods also tends to be relatively decreased as the contents of oils and fats therein decrease. It is a crucial technical problem how many sterols and/or sterol fatty acid esters are blended in foods having a small proportion of oils and fats.

With respect to the foods manufactured through an emulsification process, the sterols cannot be sufficiently emulsified due to the converted amount of the fats and oils, and thickeners and emulsion stabilizers are added in the foods. For example, as described in JP3-91460A, thickeners such as starch, guaia gum or xanthan gum are added in the low calorie mayonnaise with a low oil and fat content in order to prevent viscosity from lowering. However, the foods supplemented with the thickener become so viscous and sticky that they give unpleasant texture. For solving these problems, starch having an improved texture or a substitute of oils and fats is added (JP52-110870, JP3-39065 and JP4-36393), or polysaccharides as the thickeners are combined, or novel substances are added (JP60-41464A, JP60-49763A, JP63-173555A, JP2-227045A and JP2-227947A). Since non of these methods are not satisfactory, the amount of blending of the thickener and emulsion stabilizer has been practically reduced to be as small as possible. However, the thickener and emulsion stabilizer are forced to be added to a certain extent in foods containing a small proportion of oils and fats.

A separation type liquid dressing manufactured without applying any emulsification steps in the manufacturing process is required to maintain a homogeneity for a long period of time after shaking the dressing with no changes of the fresh texture and flavor. Adding the thickener and emulsion stabilizer into the dressing is not preferable in this case, since it becomes so viscous and sticky that its flesh feeling disappears.

In order to solve the above problems, JP2001-503623A discloses the technics, which effectively lower the blood serum cholesterol level by using stanolic fatty acid ester having more effectiveness of lowering the blood serum cholesterol level than stanol fatty acid esters, even if a small amount of stanols are added.

However, since most stanols as starting material for stanol fatty acid ester are obtained as by-product in the process of the production of pulps or by the adding hydrogen to sterols, it is not desirable to use the stanols as the starting material for foods in view of safety and consumer's taste. In addition, stanols have higher melting point than that of sterols, thereby the production process of stanol fatty acid esters is complicated and their uses are restricted.

In other words, it is necessary to regulate the degree of esterification of sterol fatty acid esters, the degree of unsaturation of fatty acids and kinds of fatty acids in view of antioxidation, reinforcement of physiological activity, delaying of crystallization, improvement of workability when adding to foods as well as improvement of solubility. In taking those factors and backgrounds into consideration, the present invention is accomplished through intensive experimentation.

### SUMMARY OF THE INVENTION

Accordingly, it is a first object of the present invention to provide foods having pleasant texture, taste and flavor by allowing the foods to contain sterol fatty acid esters composition having a degree of esterification of as high as more than 90%, whereby crystals are hardly formed during storage while enabling foods having a low proportion of oils and fats to contain a large quantity of the sterol fatty acid ester composition in addition to enabling the sterol fatty acid ester composition to exhibit an emulsifying effect.

It is a second object of the present invention to provide foods containing antioxidative sterol fatty acid esters and sterol fatty acid ester compositions that allow crystallization of the sterol and sterol fatty acid ester composition to be suppressed during storage of the foods.

It is a third object of the present invention to provide sterol fatty acid ester compositions having the performance and effects as described above.

While the foods containing the sterol fatty acid esters have been already known, crystallization of sterols was a problem since the foods was a mixture of free sterols and sterol fatty acid esters. For solving the problem, JP11-127779A discloses, for example, foods containing sterols which are esterified with the degree of esterification of 40 to 90%.

The sterols and sterol fatty acid esters thereof have been known to play an important role as starting materials of vitamin D3 and steroid hormones, and as emulsification aids and emulsification stabilizers as well as physiologically active intercellular lipids. The present invention enabled foods containing oils and fats to be obtained by adding the sterol fatty acid ester compositions, whereby free sterols are hardly crystallized during storage while exhibiting an emulsifying effect, and the foods with a low content of oils and fats display a favorable viscosity. The amount of addition of thickeners and emulsion stabilizers may be also decreased. This means that sterols with a degree of esterification of more than 90% also act as the thickeners and emulsion stabilizers.

In the present application, the sterol fatty acid esters mean esters of sterols and fatty acids. The sterol fatty acid ester composition means a mixture of esterified sterols and free sterols. Any sterols derived from a vegetable such as soybean and rape seed may be used, and the sterols include β-sitosterol, campesterol, brassica sterol and stigma sterol. In the present application, any one of β-sitosterol, campesterol, brassica sterol, stigma sterol or a mixture thereof may be used. Also vegetable oil deodrizer distillate of soybean oil, rape seed oil, palm oil, sunflower seed oil, rice bran oil, corn oil and safflower oil, or tall oil, which contain the sterols, may be used. The present invention is not limited by the above items. As a further example, high-purity sterol powder purified from vegetable oil deodorizer distillate may be used, preferably any sterols derived from vegetable such as soybean and rapeseed.

Any fatty acids including C2 to C24 saturated fatty acids and unsaturated fatty acids derived from animals or vegetables, may be used. The unsaturated fatty acids are defined as the fatty acid having at least one or plural double bonds. Examples of the saturated fatty acids include caprylic acid, decanolic acid, lauric acid, myristic acid, palmitic acid, stearic acid, arachidic acid, behenic acid, lignoceric acid, while those of the unsaturated fatty acids include palmitoleic acid, oleic acid, linoleic acid, α-linolenic acid, γ-linolenic acid and erucic acid. While linear fatty acids are usually used, branched fatty acids such as isolauric acid, 12-methylmyristic acid, 15-methylpalmitic acid, isoarachidic acid, and mixtures including one or more than two kinds thereof may be used. In the method for producing the sterol fatty acid ester composition, it is desirable to utilize enzyme as catalyst for esterification.

Sterols with a degree of esterification of more than 90% are used, although reducing a purity of sterol fatty acid ester, the value thereof is varied due to kinds of sterols and fatty acids, preferably a purity of 93% or more is used, whereby free sterols are hardly crystallized, foods having a small proportion of oils and fats may be allowed to contain a large quantity of sterol fatty acid ester composition, and to enable the amount of addition of thickeners and emulsion stabilizers to decrease to 50% or less and to have excellent texture, taste and flavor. Therefore, it is possible to utilize for any uses including foods.

The degree of esterification means the proportion (%) of the weight of esterified sterols to the total amount of sterols (free sterols and sterol fatty acid esters) and the esterified sterols in the oil phase including the sterol fatty acid ester composition. The purity of sterol fatty acid ester means the value converted into to the weight concentration (%) of the sterol fatty acid esters in the sterol fatty acid ester composition.

The reasons for regulating the degree of the esterification to be more than 90% in the present invention are as follows:
(1) As regulating the degree of esterification to be more than 90%, content of free sterols become smaller because of high purity of sterol fatty acid ester composition, thereby the possibility of blending with other optional sterols increases and the uses may be extended.
(2) The higher the degree of esterification is, the characteristics of the sterol fatty acid esters such as physiological activity, texture, taste and falavor of foods may be exhibited by optionally selecting fatty acids. In order to achieve the object, the degree of esterification of more than 90% is effective.
(3) As adjusting the degree of esterification to be more than 90%, the crystallization of sterol fatty acid esters in the foods may be delayed. Also physiological activities including lowering the blood serum cholesterol level are improved.
(4) The higher the degree of esterification becomes, the smaller the polarity, may be thereby the velocity of solution in the foods containing fats and oils increases and it is of advantageous the process of manufacturing foods.
(5) There is substantially no problem in view of the production process and cost performance because the crystallization falls within the allowable range by adjusting the degree of esterification to be more than 90%.

For the reasons (1) to (5) mentioned above, the degree of esterification of 93% or more is preferably because the above advantages further increase.

In addition, in the case of adding the sterol fatty acid ester composition having the degree of esterification of more than 90% to margarine, mayonnaise, dressing, ice cream and chocolate, the prevention of crystallization, saving production costs, improvement of flavor of foods and physiologically activity may be achieved.

Furthermore, in the case of the sterol fatty acid ester having the degree of esterification of 93% or more, fatty acids of sterol fatty acid esters affect the quality of products to be added. It is expected that the fatty acids remarkably affect the reforming of texture of mayonnaise, dressing and ice cream and it is possible to provide peculiar and fresh feeling in the mouth.

Taking the experimental fact that the higher the proportion of unsaturated fatty acids in the total fatty acids constituting the sterol fatty acid esters, the less the crystals, and the greater the degree of esterification, the less the crystals, and through intensive studies for providing quite effective and novel methods, the inventors of the present invention observed the following points: utilizing enzyme for the esterification steps between sterols and fatty acids and adjusting the degree of esterification of sterol fatty acid esters to be 93% or more, the proportion of unsaturated fatty acids in the total fatty acids to be 88% by weight or more, and the unsaturated fatty acid to have at least one or plural double bonds.

According to the present invention, it is possible to provide foods with excellent quality and stability capable of preventing free sterols and sterol fatty acid esters from crystallizing during storage of the foods while being stable against oxidation by using the sterol fatty acid esters in the foods, wherein the degree of esterification of the sterol fatty acid ester composition is 93% or more, the proportion of unsaturated fatty acids in the total fatty acids is 88% by weight or more, and the unsaturated fatty acid has at least one or plural double bonds.

The fatty acids constituting the sterol fatty acid esters in the sterol fatty acid ester composition and in the foods using the same comprise unsaturated fatty acids having at least one or plural double bonds in the molecule. Examples of the fatty acids selected from plamitoleic acid, oleic acid, linoleic acid, α-linolenic acid and γ-linolenic acid, or a mixture of at least two of them. These fatty acids may provide foods with higher market values by being endowed with the functions and effects as described above.

The sterol fatty acid ester composition according to the present invention are obtained by using an enzyme in the esterification step between sterols and fatty acids, and the degree of esterification of the sterol fatty acid ester composition is 93% or more, preferably 95% or more. The proportion of the unsaturated fatty acids in the total fatty acids constituting the sterol fatty acid esters is 88% by weight or more, preferably 88% to 94% by weight, more preferably 88% to 92% by weight and the unsaturated fatty acid contains at least one or plural double bonds.

As described above, by containing unsaturated fatty acids, it is possible to delay the crystallization velocity and to disperse sterol fatty acid ester composition in the foods in the manufacturing process of the foods, the production cost can be saved.

The reasons for adjusting the proportion of unsaturated fatty acids in the total fatty acids constituting sterol fatty acid ester according to the present invention to be 88% by weight or more are as follows: In the case of less than 88% by weight, the crystallization speed increases, and the process of removing the crystals (wintering process) is required, while in the case of 88% by weight or more, the crystallization speed decreases, the wintering process is omitted and sterol fatty acid ester in the foods are easy to disperse.

In addition, the reasons for adjusting the unsaturated fatty acid to have at least one or plural double bonds are:
(1) Vegetable oils as starting material of fatty acids generally contain one or more unsaturated fatty acids and can be obtained cheaply.
(2) By using unsaturated fatty acids, the physiological activity included in oleic acid, linoleic acid, linolenic acid and so on are maintained.
(3) In general, the higher the proportion of unsaturated fatty acids in the fatty acids constituting sterol fatty acid esters, the less the crystallization speed.
(4) The higher the unsaturation fraction of fatty acids constituting sterol fatty acid esters, the less the crystallization speed and the higher the physiological activity.
(5) The increasing of unsaturation fraction tends to accelerate oxidation, while edible oil, which is cheap, suitable for consumer's taste and useable industrially, necessarily contains unsaturated fatty acids, such as oleic acid, linoleic acid, linolenic acid.

As the results of a number of experiments carried out by the inventors of the present invention, it is found that the proportion of unsaturated fatty acids in the fatty acids constituting sterol fatty acid esters being 88% by weight or more is desirable in view of crystallization speed, oxidation, physiological activity and costs.

The reasons for adjusting the proportion of the unsaturated fatty acids in the total fatty acids constituting the sterol fatty acid esters to be less than 94% by weight are as follows: Unsaturated fatty acids of 94% by weight or more accelerate oxidation with increasing speed. Since there are a few kinds of vegetables oils and fats containing unsaturated fatty acids of 94% by weight or more, the production costs increase. In view of oxidation and production costs, unsaturated fatty acids of 94% by weight or less are advantageous.

The proportion of unsaturated fatty acids having one double bonds in the total fatty acids is 13 to 45% by weight, and/or that of unsaturated fatty acids having more than two double bonds is 45 to 75% by weight, and/or that of saturated fatty acids is 12% by weight or less; preferably that of unsaturated fatty acids having one double bonds in the total fatty acids is 13 to 45% by weight, and/or that of unsaturated fatty acids having more than two double bonds is 75% by weight or less, and/or that of saturated fatty acids is 12% by weight or less; more preferably that of oleic acid as unsaturated fatty acids having one double bonds in the total fatty acids is 15 to 30% by weight, and/or that of linoleic acid and linolenic acid as unsaturated fatty acids having more than two double bonds is 70% by weight or less, and/or palmitic acid and stearic acid as saturated fatty acids is 12% by weight or less.

As described above, by comprising unsaturated fatty acids, it is possible to decrease the crystallization speed of the sterol fatty acid composition in foods and suppress and retain the oxidation within the allowable range.

It is desirable to increase the proportion of polyunsaturated fatty acids having two double bonds, such as linoleic acid, α-linolenic acid, γ-linolenic acid, in the fatty acids in order to activate the physiological activity for lowering the blood serum cholesterol level or to decrease the crystallization speed of the sterol fatty acid esters. On the other hand, it is desirable to increase the proportion of fatty acids including saturated fatty acids such as caprylic acid, decanoic acid, laurylic acid, myristic acid, palmitic acid, stearic acid, arachidic acid and behenic acid and unsaturated fatty acids having one double bond such as palmitoleic acid, oleic acid, eicosanoic acid and erucic acid in order to decrease the oxidation speed. It is necessary for obtaining the respective functions and effects to overcome the contradiction therebetween.

Soybean oil, rape seed oil, sunflower oil, safflower oil, corn oil, sesame oil, arachis oil, rice bran oil, palm oil, olive oil, high-oleic olive oil, high-oleic sunflower oil and high-oleic corn oil as vegetable oils containing caprylic acid, decanoic acid, laurylic acid, myristic acid, palmitic acid, stearic acid, oleic acid, linoleic acid, α-linolenic acid, γ-linolenic acid, arachidic acid, eicosanoic acid, behenic acid and erucic acid are useful in view of quality control and production process. However, it is neither practical nor useful in view of production costs to control all fatty acids found in the vegetable oils and to regulate the balance among the unsaturated fatty acids within the certain range. For example, according to prior arts (WO9956558, WO20010721), which give attention to the constitution of fatty acids of sterol fatty acid esters, it is known that very few of vegetable oils having saturated fatty acids of 7% or less or 6% or less are present in olive oil, high-oleic olive oil, high-oleic sunflower oil, high-oleic corn oil and so on. From this point of view, it is difficult to utilize soybean oil, sunflower oil, safflower oil, corn oil, sesame oil, arachis oil, rice bran oil, palm oil, olive oil as they are (although they are suitable vegetable oils for consumer's taste, flavor and physiological activity) because the saturated fatty acids of 7.5% or more are contained in those oils.

In taking those problems into consideration, the inventors investigated and attempted the possible utilization of the above-mentioned oils as follows: Firstly, in the group of fatty acids such as caprylic acid, decanoic acid, laurylic acid, myristic acid, palmitic acid, palmitoleic acid, stearic acid, oleic acid, linoleic acid, α-linolenic acid, γ-linolenic acid, arachidic acid, eicosanoic acid, behenic acid and erucic acid, fatty acids containing the following saturated fatty acids and unsaturated acids above the certain range are selected and adjusted, namely, palmitic acid and stearic acid as saturated fatty acids, which are principal dietary fats and oils; oleic acid as unsaturated fatty acids having one double bond; and linoleic acid, α-linolenic acid, γ-linolenic acid as unsaturated fatty acids having more than two double bonds, are selected and prepared. In this case, the proportion of unsaturated fatty acids having one double bond in the total fatty acids should be 13 to 45% by weight, unsaturated fatty acids having more than plural double bonds should be 45 to 75% by weight and saturated fatty acids should be 12% by weight or less. As the result, it has been possible to minimize the crystallization speed, oxidation speed and production costs, to maximize physiological activity and to be much to the customers' taste. In addition, it has been advantageous in view of simplification of production process and quality control because those fatty acids are necessarily present in common vegetable oils. The sterol fatty acid ester composition according to the present invention is practically used and is of great market value.

In other words, the sterol fatty acid esters according to the prior arts are disadvantageous in view of production process and costs since kinds and containing range of constituting fatty acids are restricted. In the producing the sterol fatty acid ester composition according to the present invention, an enzymatic synthesis is used and also fats and oils having suitable fatty acid composition, which are selected in the easily available dietary fats and oils, are used, thereby is possible to produce it cheaply.

With regard to the fatty acids, while these fatty acids may be commercially available purified fatty acids, vegetable oils containing these fatty acids, for example soybean oil, sunflower oil, rape seed oil, safflower oil, corn oil, peanut oil, cotton seed oil, sesame oil and olive oil may be directly used, or purified fractions of the fatty acid after decomposition of these into fatty acids and glycerol may be used. While the fractionation methods include molecular distillation and column treatment, these are not particularly restricted. Methyl or ethyl esters of the fatty acids may be used as stating materials, although they are not preferable.

Using the above-mentioned vegetable oils for fatty acids is advantageous in view of production costs, physiological activity, the customer's taste and texture, taste and flavor.

The sterol fatty acid ester composition is produced, for example, by an esterification reaction between the sterols and fatty acids as substrates using an enzyme such as lipase or esterase having an activity for hydrolyzing the oils and fats. The fatty acid composition of the substrate is usually reflected on the fatty acid composition of the sterol fatty acid ester composition. Fractions containing the sterols are obtained in the present invention from the deodorized scum oil (the deodorized distillated oil) as a starting material, wherein triacyl glycerols contained in the deodorized scum oil are previously hydrolyzed using a chemical catalyst, followed by removing the fatty acids formed by molecular distillation. A mixture obtained by adding an arbitrary oil mainly comprising triacyl glycerol into the fraction containing the sterols obtained effect molecular distillation is used as a starting material, and the sterol fatty acid esters are synthesized under a strictly controlled condition using an enzyme having an activity for hydrolyzing oils and fats. Sterol fatty acid esters for foods are obtained by several steps of purification treatments after an enzymatic reaction for ensuring qualities suitable for foods. The Sterol fatty acid esters obtained are highly safe and inexpensive, and are almost free from degenerated fatty acids such as trans fatty acids.

Reaction conditions for the chemical synthesis are so severe that the products thereof are liable to be deteriorate or by-products may be readily formed. Accordingly, the purification process after the synthetic reaction inevitably becomes complicated with potential dangers of contamination with the by-products and reaction catalysts when the reaction products are used for foods and medicines. Therefore, an enzymatic synthesis, not a chemical synthesis, was used for esterification of fatty acids with sterols in the present invention, whereby the reaction condition becomes mild, quality of the product is hardly deteriorated, and substantially no by-products are formed.

In the esterification carried out by the enzymatic synthesis, the following processes are performed: increasing the degree of esterification to be more than 90%; regulating the proportion of unsaturated fatty acids in the total fatty acids to be 88% by weight or more; and regulating double bond of unsaturated fatty acids to be at least one or more.

Furthermore, in the esterification carried out by the enzymatic synthesis, the following processes are performed, the processes comprising; increasing the degree of esterification to be 93% or more; regulating the proportion of unsaturated fatty acids in the total fatty acids to be 88 to 94% by weight; and regulating double bond of unsaturated fatty acids to be at least one or more.

The foods comprising the sterol fatty acid ester composition according to the present invention may contain the sterol fatty acid ester composition obtained as described above. The present invention provides foods containing the sterol fatty acid ester composition, wherein the degree of esterification of the sterol fatty acid ester composition is more than 90%, preferably 93% or more; the proportion of the unsaturated fatty acids in the total fatty acids constituting the sterol fatty acid esters is 88% by weight or more, the unsaturated fatty acids in the total fatty acids preferably include palmitooleic acid, oleic acid, linoleic acid, α-linolenic acid and γ-linolenic acid, more preferably the proportion of oleic acid as unsaturated fatty acid having one double bond is 15 to 30% by weight, and/or that of linoleic acid and linolenic acids as unsaturated fatty acids having plural double bonds is 70% by weight or less, and/or that of palmitic acid and stearic acid as saturated fatty is 12% by weight or less. The proportion of unsaturated fatty acids having one or plural double bonds in the total fatty acids is 88% by weight or more, preferably 88 to 94% by weight, more preferably 88 to 92% by weight. Sterols and sterol fatty acid esters are less liable to be crystallized during storage of the foods, by using the sterol fatty acid ester composition according to the present invention satisfying the conditions above in the foods. Moreover, the foods become stable against oxidation, and foods excellent in quality and safety may be provided. Crystallization of free sterols and sterol fatty acid esters are not observed for a period of one month or more at 25°C, when the proportion of the unsaturated fatty acids in the total fatty acids constituting the sterol fatty acid ester composition according to the present invention is 92% by weight or less.

Examples of the foods containing the sterol fatty acid ester composition according to the present invention include edible oils, margarine, confectioneries, iced confectioneries, seasonings, beverages, milk and dairy products. In more detail, they include edible oils such as salad oil, olive oil, corn oil, sesame oil, soybean oil and canola oil (castor oil); margarine and related foods such as shortening and fat spread; confectioneries such as biscuit, cookie, cake, candy, jelly, chocolate, rice cracker, rice-cake cube, tablet candy and Japanese sweet; iced confectioneries such as ice cream, soft cream, sherbet and frozen yogurt; seasonings such as soy sauce, soy paste, sauce, gravy, mayonnaise and dressing; milk and dairy products such as yogurt, cheese, butter, coffee whitener, whipped cream and cow milk; beverages such as soft drinks, carbonated drink, fruit juice, milk juice, lactic acid beverage and carbonated drink. While margarine is restricted to higher grade margarine, standard margarine, processed margarine and fat spread in the Japanese Agricultural Standard (JAS), the definition of margarine is not restricted thereto in the present invention. Dressings are categorized into two types of emulsion type dressing and separation type liquid dressing, and the emulsion type dressing includes a semi-solid dressing such as low calorie mayonnaise and emulsified liquid dressing. The sterol fatty acid ester composition according to the present invention may be used for both types of dressings. An effect for lowering the proportion of the thickener, and an effect for forming a persistent dispersion after shaking the dressing may be expected in the emulsion type and separation type liquid dressings, respectively, by adding the sterol fatty acid ester composition.

The foods according to the present invention may be prepared by any methods known in the art. It is recommended to add and dissolve the sterol fatty acid ester composition in an oil phase before blending with an aqueous phase in the foods to be prepared.

The foods containing the sterol fatty acid ester composition according to the present invention may be prepared by any one of the following methods. While it is suitable to add and dissolve the sterol fatty acid ester composition according to the present invention in the oil phase before blending with the aqueous phase in the foodstuff to be prepared, the method is not restricted thereto. The sterol fatty acid ester composition according to the present invention may be directly added in foods containing no oils, or may be added together with an appropriate emulsifying agent. The foods according to the present invention may be prepared as usual foods as well as granular diets such as capsules, oral and intraintestine eutrophic diets such as thickened liquid diets, non-processed liquid diet, partially digested eutrophic diets, component controlled eutrophic diets and drink eutrophic diets. Proteins, sugars, fats, trace elements, vitamins, emulsifying agents and spices may be blended with the foods according to the present invention.

The proportion of addition of the sterol fatty acid ester composition in the foods is 1 to 50% by weight, preferably 3 to 40% by weight, and more preferably 10 to 30% by weight in the present invention. For example, since the preferable amount of the sterol fatty acid composition taken by an adult a day is 1 to 5g, the foods containing the sterol fatty acid composition is designed so as to satisfy the required amount of intake in the present invention.

### DESCRIPTION OF THE PREFERRED EMBODIMENTS

While the present invention is described hereinafter with reference to examples, the present invention by no means restricted to these examples. The term "parts" denotes "parts by weight" hereinafter

### Example 1

Added into 5000g of deodorized soybean scum oil (with a sterol content of 11.5%) was 50L of an ethanolic solution containing 0.2N of potassium hydroxide, and the mixture was stirred at 60°C for 2 hours in order to hydrolyze fatty acid esters such as triacylglycerol. The reaction solution was neutralized with 0.4N hydrochloric acid, and potassium chloride, potassium hydroxide and glycerol were removed by washing with water, followed by dehydration at 80°C under a reduced pressure. Subsequently, the residue was subjected to molecular distillation using a centrifugal molecular distillation apparatus at a reduced pressure of 1.5 Pa and a temperature of the evaporation surface of 120°C, subsequently at a reduced pressure of 1.5 Pa and a temperature of the evaporation surface of 140°C, for removing a distillation fraction mainly comprising fatty acids, thereby collecting 1,900g of a residual fraction containing sterol fatty acid esters.

Subsequently, rape seed oil (3,800g with 92% by weight of unsaturated fatty acids in the total fatty acids) was added to 1,900g of the sterol fraction collected as described above. Then, a suspension prepared by dispersing 8.3g of an enzyme powder having a lipid hydrolyzing activity (360,000 units/g, originating from genus Candida) in 2,850 ml of water was added to the oil/sterol mixture above, and sterol fatty acid esters were synthesized at 40°C for 24 hours with stirring. Subsequently, the enzyme was deactivated by heating at 80°C with stirring for 30 minutes. The reaction mixture was washed with warm water, dehydrated at 80°C under a reduced pressure, and filtered with stirring by adding 11.5g of diatoms earth for removing enzyme proteins.

Subsequently, unreacted fatty acids and sterols were removed as distillation fractions by molecular distillation at a reduced pressure of 1.5 Pa and at a temperature of 150°C, then at reduced pressure of 1.5 Pa and at a temperature of 210°C, using a centrifugal molecular distillation apparatus. Then, 5% of activated clay and adsorbent, and five parts by volume of hexane relative to the molecular distillation residue containing sterol fatty acid esters were added, and the mixture was stirred at room temperature for 30 minutes, followed by removing the activated clay and adsorbent, on which pigments are adsorbed, by filtration. Finally, the residue was steam-distilled for 1 hour at a reduced pressure of 500 Pa and distillation temperature of 130°C using a batch type steam distillation apparatus, thereby obtaining 790g of sterol fatty acid esters from which odor components have been removed.

The odorless and pale yellow colored sterol fatty acid ester composition obtained had a degree of esterification of 99%, and the content of the unsaturated fatty acid in the total fatty acids was 92% by weight.

### Example 2

Sterol fatty acid ester compositions (with a degree of esterification of 99%) in which the fatty acids are composed of palmitic acid (C16:0) only and oleic acid (C18:1) only, respectively, were prepared with reference to Example 1. After mixing these sterol fatty acid ester compositions with each other in respective proportions shown in Table 1, each mixture was heated at 80°C together with soybean oil to melt the crystals of the sterol fatty acid ester completely. Then, the sterol fatty acid ester composition was added to soybean oil so that the proportion of the ester becomes 10% by weight as converted into the proportion of sterols, and was mixed with a vortex mixer for 1 minute. The mixture was heated at 80°C for 30 minutes, followed by allowed to cool to room temperature (25°C). After allowing the mixture to stand still for additional 6 hours at 25°C, crystallization of the sterol fatty acid ester composition was observed with the naked eye.

The results are shown in Table 1. No crystallization of the sterol fatty acid ester composition was observed in the sample Nos. 1 to 6, wherein the fatty acids comprise 87% by weight or more of the unsaturated fatty acid. Few amount of crystals of the sterol fatty acid ester were observed in the sample No. 7, and soybean oil remained transparent with no turbidity except a trace amount of white precipitates at the bottom. These results suggests that crystallization tends to be suppressed as the content of the unsaturated fatty acid constituting the sterol fatty acid ester increases.

No crystallization was observed in the sample Nos. 1 to 4 after allowing them to stand for one month at 25°C, wherein the sterol fatty acid ester compositions comprise 92% by weight or more of the unsaturated fatty acid in the total fatty acids.

**TABLE 1**

| SAMPLE | FATTY ACID CONSTITUTING STEROL FATTY ACID ESTER (% by weight) | | EXTENT OF CRYSTALLIZATION |
|---|---|---|---|
| | C16:0 | C18:1 | |
| 1 | 0 | 100 | - |
| 2 | 1 | 99 | - |
| 3 | 5 | 95 | - |
| 4 | 7.5 | 92.5 | - |
| 5 | 10 | 90 | - |
| 6 | 12.5 | 87.5 | - |
| 7 | 15 | 85 | -+ |
| Evaluation of the extent of crystallization; small: +, very small: -+, no crystallization:- | | | |

### Example 3

Sterol fatty acid ester compositions (with a degree of esterification of 99%) in which the fatty acids are composed of palmitic acid (C16:0) only and linoleic acid (C18:2) only, respectively, were prepared with reference to Example 1. After mixing these sterol fatty acid ester compositions with each other in respective proportions shown in Table 2, each mixture was heated at 80°C together with soybean oil to melt the crystals of the sterol fatty acid ester completely. Then, the sterol fatty acid ester composition was added to soybean oil so that the proportion of the ester becomes 10% by weight as converted into the proportion of sterols, and was mixed with a vortex mixer for 1 minute. The mixture was heated at 80°C for 30 minutes, followed by allowed to cool to room temperature (25°C). After allowing the mixture to stand still for additional 6 hours at 25°C, crystallization of the sterol fatty acid ester composition was observed with the naked eye.

The results are shown in Table 2. No crystallization of the sterol fatty acid ester composition was observed in the sample Nos. 1 to 6, wherein the fatty acids comprise 87% by weight or more of the unsaturated fatty acid. Few amount of crystals of the sterol fatty acid ester were observed in the sample No. 7, and soybean oil remained transparent with no turbidity except a trace amount of white precipitates at the bottom. These results suggests that crystallization tends to be suppressed as the content of the unsaturated fatty acid constituting the sterol fatty acid ester increases.

No crystallization was observed in the sample Nos. 1 to 4 after allowing them to stand for one month at 25°C, wherein the sterol fatty acid ester compositions comprise 92% by weight or more of the unsaturated fatty acid in the total fatty acids.

**TABLE 2**

| SAMPLE | FATTY ACID CONSTITUTING STEROL FATTY ACID ESTER (% by weight) | | EXTENT OF CRYSTALLIZATION |
|---|---|---|---|
| | C16:0 | C18:2 | |
| 1 | 0 | 100 | - |
| 2 | 1 | 99 | - |
| 3 | 5 | 95 | - |
| 4 | 7.5 | 92.5 | - |
| 5 | 10 | 90 | - |
| 6 | 12.5 | 87.5 | - |
| 7 | 15 | 85 | -+ |
| Evaluation of the extent of crystallization; small: +, very small: -+, no crystallization: - | | | |

### Example 4

A sterol fatty acid ester composition (with a degree of esterification of 99%) in which the fatty acids are composed of 94% by weight of unsaturated fatty acid was prepared with reference to Example 1. The unsaturated fatty acids were composed of 5% by weight of palmitooleic acid, 34% by weight of oleic acid, 45% by weight of linoleic acid, 6% by weight of α-linolenic acid, and the saturated fatty acids were composed of 4% by weight of palmitic acid and 1% by weight of stearic acid. Soybean oil was added to each sterol fatty acid ester composition obtained in a proportion shown in Table 3, and was mixed for 1 minute with a vortex mixer. After heating each mixture at 80°C for 30 minutes, it was allowed to cool to room temperature (25°C). After allowing the mixture to stand still for additional 24 hours at 25°C, crystallization of the sterol fatty acid ester composition was observed with the naked eye.

The results are shown in Table 3. No crystallization of the sterol fatty acid ester composition was observed in the sample Nos. 1 to 7, wherein the content of the sterol fatty acid esters was 50% by weight or less as converted into the sterols. The sample No. 8 containing 55% by weight of the sterol fatty acid ester as converted into sterols showed no turbidity in soybean oil while remaining transparent except few white precipitates at the bottom.

**TABLE 3**

| SAMPLE | STEROL FATTY ACID ESTER (% by weight as converted into sterols) | EXTENT OF CRYSTALLIZATION |
|---|---|---|
| 1 | 1% | - |
| 2 | 3% | - |
| 3 | 5% | - |
| 4 | 10% | - |
| 5 | 30% | - |
| 6 | 40% | - |
| 7 | 50% | - |
| 8 | 55% | -+ |
| Evaluation of the extent of crystallization; small: +, very small: -+, no crystallization: - | | |

### Example 5

A sterol fatty acid ester composition (degree of esterification, 99%) comprising 89% by weight of unsaturated fatty acids in the total fatty acids constituting sterol fatty acid ester composition was prepared with reference to Example 1. The unsaturated fatty acids were composed of 28% by weight of oleic acid, 55% by weight of linoleic acid and 6% by weight of α-linolenic acid, and the saturated fatty acids were composed of 8% by weight of palmitic acid and 3% by weight of stearic acid. The sterol fatty acid ester composition obtained was used for an oxidation stability test.

For the oxidation stability test, 5g of the sterol fatty acid ester composition was placed in a seal type test tube, and the composition was sealed after purging the test tube with nitrogen. After allowing the test tube to stand still for 7 days at 60°C, POV (peroxide value) of the sample was measured. The POV was determined by the method according to AOCS, Cd86-90.

The POV obtained was 0.8 and 2.4 on the first day and on the 7th day, respectively, showing that POV were suppressed from increasing even under a sever condition of 60°C. This means that the sterol fatty acid ester composition according to the present invention has excellent oxidation stability.

### Example 6

Mixed and processed as an aqueous composition were 20 parts of vinegar (acetic acid content 5%), 4 parts of sugar, 30 parts of water, 0.5 parts of starch, 2 parts of table salt, 0.4 parts of sodium L-glutamate, 0.1 parts of white pepper, 0.1 parts of mustard and 1.4 parts of vegetable extract. A premix comprising 10 parts of rape seed oil and 10 parts of sterol fatty acid ester composition (with a degree of esterification of 99%) was added to the aqueous composition, and mixed for 5 minutes with stirring with a mixer. Another 20 parts of rape seed oil was added to the preparation to form a dressing composition. This dressing composition was allowed to pass through an emulsifier (colloid mill), thereby obtaining an emulsified liquid dressing. The dressing had a viscosity (20°C) of 3000 cps that is almost the same as the viscosity of the conventional dressing irrespective of a low content of starch in the dressing with neither viscous nor sticky feeling peculiar to the thickeners.

### Comparative Example 1

Mixed and processed as an aqueous composition were 20 parts of vinegar (acetic acid content 5%), 4 parts of sugar, 30 parts of water, 3 parts of starch, 2 parts of table salt, 0.4 parts of sodium L-glutamate, 0.1 parts of white pepper, 0.1 parts of mustard and 1.4 parts of vegetable extract. Added to the aqueous composition was 22.5 parts of rape seed oil, and was mixed with stirring for 5 minutes using a mixer. Another 20 parts of rape seed oil was added to the preparation to form a dressing composition. This dressing composition was allowed to pass through a colloid mill, thereby obtaining an emulsified liquid dressing. The dressing had a viscosity (20°C) of 3200 cps, and showed sticky feeling peculiar to thickeners with unfavorable flavor.

### Example 7

Mixed and processed as an aqueous composition were 20 parts of vinegar (acetic acid content 5%), 5 parts of sugar, 30 parts of water, 4 parts of table salt, 0.4 parts of sodium L-glutamate, 0.1 parts of white pepper, 0.2 parts of mustard and 1.4 parts of vegetable extract. Mixed as an oil phase were 35 parts of rape seed oil and 5 parts of sterol fatty acid ester composition (with a degree of esterification of 99%). The aqueous phase and oil phase were mixed in a volume ratio of 4 : 6 to prepare a separation type liquid dressing. No sterols were crystallized after storing the dressing in a refrigerator (4°C). Dispersion of the dressing by shaking was evaluated as in Table 4, showing that the oil phase remains dispersed in the aqueous phase at 30 minutes after shaking.

### Comparative Example 2

Rape seed oil was mixed with the aqueous composition obtained as described in Example 6 in a volume ratio of 4 : 6 to prepare a separation type dressing. Dispersion of the dressing by shaking was also evaluated as in Table 4, showing that the oil phase is completely separated from the aqueous phase at 5 minutes after shaking.

**TABLE 1**

| DISPERSION OF DRESSING | | | | |
|---|---|---|---|---|
| | IMMEDIATELY AFTER | 1 MINUTES AFTER | 10 MINUTES AFTER | 30 MINUTES AFTER |
| EXAMPLE 2 | +++ | +++ | ++ | ++ |
| COMPARATIVE EXAMPLE 2 | + | + | - | - |
| - : perfect separation | | | | |
| + : partial floating of oil phase | | | | |
| ++ : dispersion | | | | |
| +++ : perfect dispersion | | | | |

### Example 8

Mixed and processed as an aqueous composition were 10 parts of vinegar (acetic acid content 5%), 2.5 parts of sugar, 38 parts of water, 2 parts of starch, 1 part of table salt, 0.4 parts of sodium L-glutamate, 0.1 parts of white pepper and 1 parts of mustard. A premix comprising 20 parts of rape seed oil and 10 parts of sterol fatty acid ester composition (with a degree of esterification of 99%) was poured into the aqueous composition with stirring for thorough mixing. Then, the mixture was sufficiently emulsifies with a colloid mill to obtain a low calorie mayonnaise. The mayonnaise had a viscosity (20°C) of 50000 cps that is almost the same as the viscosity of the conventional mayonnaise, and neither viscous nor sticky feeling peculiar to thickeners was felt by reducing the content of starch to half of the conventional products. No sterol crystals were found after storage in a refrigerator (4°C) overnight.

### Comparative Example 3

Rape seed oil (30 parts) was poured into 70 parts of an aqueous composition prepared by blending as in Example 8 with thorough mixing. The mixture was then sufficiently emulsified with a colloid mill to obtain a low calorie mayonnaise. The mayonnaise had a lower viscosity (20°C) of 32000 cps as compared with the mayonnaise obtained in Example 8.

### Example 9

Thoroughly stirred at 60°C were 60 parts of hydrogenated rape seed oil (melting point 36°C), 17.7 parts of cotton seed oil, 20 parts of sterol fatty acid ester composition (with a degree of esterification of 99%) and 0.3 parts of polyglycerin fatty acid ester, and the oil composition was used as a thickened sterol fatty acid ester. An aqueous composition was prepared by adding a small amount of whey proteins, a flavoring agent and citric acid followed by adjusting pH at 4.5. The thickened sterol fatty acid ester (40 parts) and aqueous composition (60 parts) were mixed at 60°C. The mixture was emulsified, quenched and crystallized thereafter by a method known in the art, thereby obtaining a 40% fat spread. No crystals of sterols were formed in the product obtained. No problems were encountered by decreasing the proportion of the emulsifying agent that has been usually blended in a proportion of 1 to 3 parts, enabling a good product to be obtained.

### Example 10

Thoroughly stirred at 60°C were 60 parts of hydrogenated rape seed oil (melting point 36°C), 17.7 parts of cotton seed oil, 20 parts of a sterol fatty acid ester composition (degree of esterification 99%; unsaturated fatty acids were composed of 42% by weight of oleic acid, 41% by weight of linoleic acid and 5% by weight of α-linolenic acid, and saturated fatty acids comprise 4% by weight of palmitic acid and 7% by weight of stearic acid) and 0.3 parts of polyglycerin fatty acid ester, and the oil composition was used for the thickened sterol fatty acid ester composition. A small quantity of whey proteins, a flavoring agent and citric acid were added in 20 parts of water to prepare an aqueous composition, which was adjusted to pH 4.5. The sterol fatty acid ester composition (40 parts) and the aqueous composition (60 parts) were mixed at 60°C, followed by emulsification and quenching to obtain a 40% fatty acid spread.

Ten examiners were engaged in sensing tests with respect to the spread according to the present invention. The sensing test items were color, external appearance, taste, smell, the feel to the tongue, aftertaste and flavor.

The examiners evaluated that the spread according to this example has an emulsified layer with fine and dense texture, and feeling to the tongue and aftertaste were also excellent. With respect to the external appearance, the spread has a glossy surface with no crystallization of the sterol fatty acid ester composition. The spread was concluded to be a good product with high market values.

The spread according to this example was stored in a refrigerator immediately after the production. No crystals of the sterol fatty acid ester composition were observed in the spread after 3 days' storage in a refrigerator, and the spread remained to exhibit the same texture, taste and flavor as those before refrigeration.

### Example 11

Blended as an aqueous composition were 20 parts of vinegar (acetic acid content 5%), 5 parts of sugar, 30 parts of water, 4 parts of table salt, 0.4 parts of sodium L-glutamate, 0.1 parts of white pepper, 0.2 parts of mustard and 2.5 parts of vegetable extract. An oil composition was prepared by mixing 35 parts of rape seed oil and 5 parts of a sterol fatty acid ester composition (degree of esterification of 99%; unsaturated fatty acids were composed of 42% by weight of oleic acid, 43% by weight of linoleic acid and 5% by weight of α-linolenic acid,and saturated fatty acids were composed of 3% by weight of palmitic acid and 7% by weight of stearic acid). A separation type liquid dressing was obtained by blending the aqueous composition and oil composition in a proportion of 4 : 6.

The separation type liquid dressing obtained in this example was subjected to sensing tests by 10 examiners on one day after the production of the dressing. The sensing test items were color, external appearance, taste, smell, feeling to the tongue, aftertaste and flavor.

The examiners evaluated that the separation type liquid dressing according to this example was excellent in texture with good feeling to the tongue and after taste. The external appearance was also evaluated to be good since the interface between the aqueous phase and oil phase was clear in the separation state of the dressing, and no crystals of the sterol fatty acid ester composition were found in the product. In summary, the dressing was evaluated to be a good separation type liquid dressing with high market values.

As hitherto described, the present invention provides oil and fat based foods in which crystals are hardly formed during storage. A lot of sterol fatty acid esters may be incorporated in the foods having a low content of oils and fats, and the foods exhibit a good emulsifying effect, thereby enabling foods containing oils and fats having excellent texture and taste to be obtained.

The present invention also provides foods containing a sterol fatty acid ester composition prepared by utilizing an enzyme in an esterification step between sterols and fatty acids. Consequently, the present invention provides foods containing the sterol fatty acid ester composition, wherein the degree of esterification of the sterols is 93% or more, the proportion of unsaturated fatty acids in the total fatty acids is 88% by weight or more, and the unsaturated fatty acid group/moiety has at least one double bond.

According to the present invention, the amount of crystals formed during storage of the foods may be evidently decreased.

The content of free sterols having a low solubility in oils and fats are restricted to be as small as possible while enabling an oxidation stable sterol fatty acid ester composition to be obtained, since a sterol fatty acid ester composition with a degree of esterification of 93% or more is used in the present invention while using unsaturated fatty acids, such as oleic acid having more than one double bond, as fatty acids constituting the ester composition.

## Claims

1. Foods containing a sterol fatty acid ester composition with the degree of esterification of sterols of more than 90%.

2. Foods containing a sterol fatty acid ester composition obtained by utilizing an enzyme in an esterification step between sterols and fatty acids, wherein the degree of esterification of sterols is more than 90%.

3. Foods containing a sterol fatty acid ester composition obtained by utilizing an enzyme in an esterification step between sterols and fatty acids, wherein the degree of esterification of the sterol fatty acid ester composition accounts for 93% or more, the proportion of unsaturated fatty acids in the total fatty acids constituting the sterol fatty acid esters accounts for 88% by weight or more, and the unsaturated fatty acids have at least one or plural double bonds.

4. Foods containing a sterol fatty acid ester composition obtained by utilizing an enzyme in an esterification step between sterols and fatty acids, wherein the degree of esterification of the sterol fatty acid ester composition accounts for 93% or more, the proportion of unsaturated fatty acids in the total fatty acids constituting the sterol fatty acid esters accounts for 88% to 94% by weight, and the unsaturated fatty acids have at least one or plural double bonds.

5. Foods containing a sterol fatty acid ester composition according to Claims 1 to 4, wherein a food contains the sterol fatty acid esters composition at least 1% by weight or more.

6. The foods containing a sterol fatty acid ester composition according to Claims 1 to 4, wherein the unsaturated fatty acid having at least one double bond in the sterol fatty acid molecule is at least one of the fatty acids selected from palmitoleic acid, oleic acid, linoleic acid, α-linolenic acid, γ-linolenic acid, erucic acid or a mixture of two or more of them.

7. The foods containing a sterol fatty acid ester composition according to Claims 1 to 6, wherein the material of the fatty acid constituting the sterol fatty acid esters is derived from a vegetable oil.

8. The foods containing a sterol fatty acid ester composition according to Claims 1 to 7, wherein a food is selected from an edible oil, margarine, confectioneries, iced confectioneries, seasonings, beverages, milk and dairy products.

9. A sterol fatty acid ester composition obtained by utilizing an enzyme in an esterification step between sterols and fatty acids, wherein the degree of esterification of sterols is more than 90%.

10. A sterol fatty acid ester composition obtained by utilizing an enzyme in an esterification step between sterols and fatty acids, wherein the degree of esterification of the sterol fatty acid ester composition accounts for 93% or more, the proportion of unsaturated fatty acids in the total fatty acids constituting the sterol fatty acid esters accounts for 88% by weight or more, and the unsaturated fatty acids have at least one or plural double bonds.

11. A sterol fatty acid ester composition obtained by utilizing an enzyme in an esterification step between sterols and fatty acids, wherein the degree of esterification of the sterol fatty acid ester composition accounts for 93% or more, the proportion of unsaturated fatty acids in the total fatty acids constituting the sterol fatty acid esters accounts for 88% to 94% by weight, and the unsaturated fatty acids have at least one or plural double bonds.

12. A process for producing a sterol fatty acid ester composition obtained by utilizing an enzyme in an esterification step between sterols and fatty acids, the process comprising the step of increasing the degree of esterification of sterols to be more than 90%.

13. A process for producing a sterol fatty acid ester composition obtained by utilizing an enzyme in an esterification step between sterols and fatty acids, the process comprising the steps of:
regulating the degree of esterification of the sterol fatty acid ester composition to become 93% or more,
regulating the proportion of unsaturated fatty acids in the total fatty acids constituting the sterol fatty acid esters to become 88% by weight or more, and
regulating the unsaturated fatty acids to have at least one or plural double bonds.

14. A process for producing a sterol fatty acid ester composition obtained by utilizing an enzyme in an esterification step between sterols and fatty acids, the process of the steps of:
adjusting the degree of esterification of the sterol fatty acid ester composition to become 93% or more,
adjusting the proportion of unsaturated fatty acids in the total fatty acids constituting the sterol fatty acid esters to become 88% to 94% by weight, and
adjusting the unsaturated fatty acids to have at least one or plural double bonds.
